# EUROPEAN PATENT APPLICATION

(11) **EP 3 998 246 A1**
(43) Date of publication of application: **18.05.2022**
(21) Application number: 20929908.0
(22) Date of filing: 11.11.2020
(51) Int. Cl.: C04B 35/58, C04B 35/622, B01J 27/24, B01J 35/06, C07C 1/02, C07C 9/04

(54) **NITRIDE HIGH-ENTROPY CERAMIC FIBER, PREPARATION METHOD THEREOF, AND APPLICATION THEREOF**

(30) Priority: 09.04.2020 CN 202010273050
(71) Applicant: INSTITUTE OF CHEMISTRY, CHINESE ACADEMY OF SCIENCES, Beijing 100190 (CN)
(72) Inventor: ZHAO, Tong, Beijing 100190 (CN); YE, Li, Beijing 100190 (CN); LI, Wei, Beijing 100190 (CN); SUN, Yanan, Beijing 100190 (CN)
(74) Representative: Isarpatent
(86) International application number: PCT/CN2020/127990
(87) International publication number: WO 2021/203695

(57) **Abstract**

Disclosed are a high-entropy nitride ceramic fiber, and a preparation method and use thereof. The high-entropy ceramic fiber comprises Ti, Hf, Ta, Nb, and Mo; the high-entropy nitride ceramic fiber presents single crystal phase, and each of the elements are uniformly distributed at molecular level. The preparation method of the high-entropy ceramic fiber comprises: mixing a high-entropy ceramic precursor comprising the target metal elements, a spinning aid, and a solvent uniformly to prepare a precursor spinning solution, followed by working procedures of spinning, pyrolyzation, and nitriding to prepare the high-entropy nitride ceramic fiber. The high-entropy nitride ceramic fiber can be used in photocatalysis process of carbon dioxide to prepare methane.

## Description

### Field of the Invention

The present invention belongs to the field of materials and relates to high-entropy ceramics, particularly to a high-entropy nitride ceramic fiber and a preparation method and use thereof.

### Background of the Invention

High-entropy ceramics are single-phase solid solutions composed of at least five elements with the content of each of the elements ranging from 5% to 35%. There has been still little research on high-entropy ceramics so far. At present, high-entropy ceramics are present only in the forms of powder, block, or coating. Research on the properties of high-entropy ceramics is also limited to a few fields.

High-entropy nitride ceramics are single crystal ceramics composed of at least five metal elements and nitrogen, and research now mainly focuses on preparation of high-entropy nitride ceramic coatings or powders. In 2012, V. Braic, *et al.* deposited (TiZrNbHfTa)N coating on the surface of stainless steel by physical vapor deposition, which increased surface hardness to 33 GPa. The surface hardness, compared with that of conventional metal coatings, increased by more than three times (V. Braic, Alina Vladescu, "Nanostructured multi-element (TiZrNbHfTa)N and (TiZrNbHfTa)C hard coatings", Surface & Coatings Technology, 211(2012): 117-121). However, the physical vapor deposition method has high requirements for equipment, and can only be used to prepare single two-dimensional materials such as coatings, which limits the application of the high-entropy nitrides. In 2018, East China University of Science and Technology, and the University of Tennessee and Oak Ridge National Laboratory in the US jointly developed a method of preparing high-entropy nitride ceramic powders. The method used metal chlorides and urea as raw materials to prepare single crystal (VCrNbMoZr) N nano-powders at 800 °C, which showed potential application value in supercapacitors (Tian JIN, Xiaohan SANG, "Mechanochemical-Assisted Synthesis of High-Entropy Metal Nitride via a Soft Urea Strategy", 2018(30):1707512). However, there are problems in the preparation of devices with powder materials, such as easy falling off, inhomogeneous dispersion, and the like.

Fibers are one-dimensional materials characterized by a small size, a high specific surface area, etc. Due to the limitation of dimension, their physical and chemical properties would change significantly compared with powder, block, and coating, and have wide application prospects in the fields of electronic information, energy catalysis, and so forth.

Photocatalysis is a type of reaction that takes ultraviolet or visible lights as the light sources and semiconductors as the catalysts to catalyze chemical reactions, such as the degradation of organic pollutants, the decomposition of water to hydrogen, the conversion of CO₂, or the like. At present, photocatalysts are mainly in the form of powders, which are difficult to separate from catalytic raw materials and products and thus difficult to recycle.

Due to the macrography form of cotton or cloth, photocatalytic fibers can be conveniently separated from catalytic raw materials and products, which are conducive to the recovery and reuse of the catalysts. Therefore, the photocatalytic fibers have become a hot development field of photocatalysts.

There has been no report on high-entropy nitride ceramic fibers so far, let alone any precedent of applying high-entropy nitride ceramics to the field of photocatalysis.

In view of this, the present invention is proposed.

### Summary of the Invention

The technical problem to be solved by the present invention is to provide a high-entropy nitride ceramic fiber and a preparation method thereof so as to overcome the shortcomings in the prior art. In this method, green fibers are prepared by blowing spinning, electrospinning, or thread throwing, and the green fibers are pyrolyzed and nitrided to prepare the high-entropy nitride ceramic fiber. The present invention overcomes the limitation that high-entropy nitride ceramics can only exist in the form of powders, blocks, or coatings, and expands the existing forms of high-entropy ceramics to the field of fibers and the use scope thereof to the field of photocatalysis.

In order to solve the above technical problem, the basic concept of a technical solution adopted by the present invention is as follows.

The present invention provides a high-entropy nitride ceramic fiber comprising Ti, Hf, Ta, Nb, and Mo, wherein the high-entropy nitride ceramic fiber presents a single crystal phase, wherein each of the elements therein is uniformly distributed at molecular level.

In a further embodiment of the preset invention, each metal elements in the high-entropy ceramic fiber occupy 5-35 molar percentage of the total metal elements. Preferably, all the metal elements are equimolar.

The high-entropy ceramic fiber also contain nitrogen element; and the molar quantity of nitrogen equals to the total molar quantity of Ti, Hf, Ta, Nb, and Mo.

In the above embodiment, the fiber is formed by dense packing particles, which has smooth surface and a length diameter ratio no less than 50.

In the above high-entropy ceramic fiber, particularly, the molar ratio of metal elements can be Ti: Hf: Ta: Nb: Mo = 8-30: 5-35: 5-35: 10-30: 5-35 or Ti: Hf: Ta: Nb: Mo = 10-20: 10-30: 3-35: 15: 10-30, and more particularly, Ti: Hf: Ta: Nb: Mo = 10:35:35:10:10, 15:30:35:15:5, 20:30:30:15:5, 30:5:35:15:15, 20:15:5:30:30, 8:12:30:15:35, 10:10:30:20, or 30:5:35:15:15.

The present invention further provides a method for preparing the high-entropy nitride ceramic fiber as described above, comprising: mixing high-entropy ceramic precursor which comprises target metal elements, spinning aid, and solvent uniformly, to form a precursor spinning solution, followed by spinning, pyrolyzing, and nitriding, wherein the target metals are selected from the group consisting of Ti, Hf, Ta, Nb, and Mo.

That is, the high-entropy ceramic precursor contain Ti, Hf, Ta, Nb, and Mo, spinning aid, and solvent are uniformly mixed to form the precursor spinning solution, and then the precursor spinning solution is subjected to procedures of spinning, pyrolyzing, and nitriding to prepare the high-entropy nitride ceramic fiber.

According to the above preparation method, the preparation steps of the high-entropy ceramic precursor comprise:
step (1) obtaining metal alkoxide complexes:
   adding dropwise complexing agent into metal alkoxides M(OR)ₙ which comprise the target metal element, followed by stirring for 0.1-5 hours to obtain the metal alkoxide complexes;
step (2) cohydrolysis:
   uniformly mixing the selected metal alkoxide complexes which comprise different metal elements prepared according to step (1), into which a mixture of water and a monohydric alcohol is slowly added dropwise, followed by refluxing for 1-5 hours, and atmospheric distillation to obtain a metal alkoxide copolymer;
step (3) preparing the precursor:
   uniformly mixing the metal alkoxide copolymer prepared in step (2) with allyl-functional novolac resin, raising the temperature to 50-90 °C (which particularly can be 80 °C) for 0.5-4 hours (which particularly can be 1-3 hours), and cooling to obtain the high-entropy ceramic precursor.

According to the above preparation method, in the precursor spinning solution, a mass ratio of the high-entropy ceramic precursor to the spinning aid to the solvent is 1: 0.1-1: 5-20, preferably 1: 0.2-0.5: 5-10 and more particularly 1: 0.33-0.4: 5-9.5, 1: 0.33-0.4: 5-9.3, 1: 0.33-0.4: 5-9.67, 1: 0.33-0.4: 5-10, 1: 0.33-0.4: 5-20, 1: 0.33-0.4: 5-5.3, or 1: 0.33-0.4: 5-6.2.

In the above embodiment, the spinning precursor solution can be prepared or mixed by other technical means, including, but not limited to, stirring, ultrasound, etc.
According to the above preparation method, the molar ratio of the metal alkoxides to the complexing agent in step (1) is 1: (0.15-0.5) n;
the complexing agent is acetylacetone and/or ethyl acetoacetate;
when M in the metal alkoxides is Ti or Hf, n is 4;
when M in the metal alkoxides is Nb, Ta, or Mo, n is 5; and
in the M(OR)ₙ, R is at least one selected from the group consisting of C1-C6 alkyl and C1-C6 alkoxy, particularly at least one selected from the group consisting of C1-C4 alkyl and C1-C4 alkoxy, and more particularly at least one selected from the group consisting of ethyl, ethylene glycol diethyl ether group, i-Pr, - Pr, and - CH₂CH₂OCH₃.

In the above embodiment, the inventors of the present invention found that the reactivity of metal alkoxides and complexing agent is greatly affected by the types of metal elements. If the complexing agents are added at similar proportions, although alkoxide complexes can be formed, the hydrolysis rate of the resulting alkoxide complexes will be affected. So improper amounts of the complexing agents could lead to mismatch of the cohydrolysis rate of the alkoxide complexes, thus result in non-homogeneous element distribution of metals in high-entropy ceramic precursor. By adopting the molar ratio of metal alkoxide to complexing agent provided in the present application, however, the above problem can be addressed and stable system can be formed in cohydrolysis process, which is conducive to the formation of the high-entropy ceramic fiber.

More particularly, when M in the metal alkoxide is Hf, Nb, Ta, or Mo, the alkoxide is prepared as follows: dispersing the metal salt MClₙ or M(NO₃)ₙ in the solvent, adding dropwise a monohydric alcohol at a temperature ranging from -10 °C to 5 °C, and then adding dropwise triethylamine, followed by refluxing for 1-5 hours and filtration to obtain the metal alkoxide solution,
wherein the molar ratio of the metal salt to the monohydric alcohol to triethylamine is 1: (1-2)n: (1-1.5)n, particularly, the molar ratio of the metal salt to the monohydric alcohol to triethylamine is 1:4-10:4-7, and more particularly 1:4:4, 1:5:5, 1:6:5, 1:6:6, 1:8:7, or 1:10:6;
wherein the solvent is one or more selected from the group consisting of n-hexane, n-heptane, toluene, xylene, ethylene glycol dimethyl ether, ethylene glycol diethyl ether, and tert-butyl methyl ether; and
wherein the monohydric alcohol is one or more selected from the group consisting of methanol, ethanol, isopropanol, n-propanol, n-butanol, isobutanol, ethylene glycol monomethyl ether, and ethylene glycol ethyl ether.

According to the above preparation method, the molar ratio of water to the total metals in step (2) is 0.8-1.3:1, particularly1-1.2:1, and the mass ratio of the monohydric alcohol to water is 3-8:1, particularly 5:1.

The monohydric alcohol is one or more selected from the group consisting of methanol, ethanol, isopropanol, n-propanol, n-butanol, isobutanol, ethylene glycol monomethyl ether, and ethylene glycol ethyl ether.

The temperature of cohydrolysis is in a range from room temperature to 90 °C. The time of cohydrolysis is 2 hours particularly.

In the above embodiment, the ratio of the alcohol to water provided by the present invention is obtained based on consideration of a mixture of the metal alkoxide complexes having different reaction activities. As a result, the reaction activities of a variety of metal alkoxide complexes tend to be similar during cohydrolysis, thereby obtaining a precursor with homogenous distribution of respective elements at molecular level.

According to the above preparation method, the ratio of the total molar quantity of the metal elements in the metal alkoxide copolymer to the mass quantity of allyl phenolic in step (3) is 1 mol: 18-20 g, in particular 1 mol: 19.5 g.

In the above preparation method, different metals have different molar masses. Thus, it is inconvenient to calculate all the metals based on the mass. The present invention herein calculates the metals according to the total molar quantity in the metal alkoxide copolymer. And the allyl-functional novolac resin is a kind of heteropolymer, which is unsuitable to be measured by molar quantity. Therefore, the ratio of molar quantity to mass quantity is adopted for expression.

According to the above preparation method, the nitriding process includes: nitriding the pyrolyzed fiber in ammonia atmosphere at a temperature of 600-1000 °C, particularly 700 °C -900 °C or 800 °C for 0.5-5 hours, particularly 2-3 hours or 2.5 hours.

According to the above preparation method, the spinning aid is one or more selected from the group consisting of polymethylmethacrylate, polyvinyl acetate, polyvinyl butyral, and polyvinylpyrrolidone; and the solvent is one or more selected from the group consisting of ethanol, acetone, n-propanol, ethylene glycol monomethyl ether, and N, N-dimethylformamide.

In the above embodiment, the spinning aid helps to improve the rheological properties of the solution, such as viscosity, dispersion homogeneity, and stability. Conventional spinning aids in the art can be used, preferably one or more selected from the group consisting of polymethylmethacrylate, polyvinyl acetate, polyvinyl butyral, and polyvinylpyrrolidone, e.g., a mixture of polymethylmethacrylate and polyvinyl acetate at any proportions, or a mixture of polyvinyl acetate and polyvinyl butyral at any proportions, or a mixture of polyvinyl butyral and polyvinyl pyrrolidone at any proportions.

In the above embodiment, the solvent used can be any solvent, and a selected solvent is beneficial to the dissolution and dispersion of raw materials, preferably one or more selected from the group consisting of ethanol, acetone, n-propanol, ethylene glycol monomethyl ether, tert-butyl methyl ether, and N, N-dimethylformamide.

According to the above preparation method, the pyrolyzing process comprises raising the temperature to 500-600 °C or 550 °C at a heating rate of 0.5-5 °C /min in an inert atmosphere and holding for 2-4 hours. Particularly, the heating rate is 1-2 °C /min or 1.5 °C /min. The temperature is kept particular for 3 hours.

In the above embodiment, the inert atmosphere is one or more selected from the group consisting of nitrogen, argon, and helium.

According to the above preparation method, the spinning is one selected from the group consisting of blowing spinning, electrospinning, and centrifugal spinning.

In the above embodiment, the spinning preferably adopts the blowing spinning technology. Blowing spinning is performed at the conditions of: a spinning pressure of 0.02-0.2 MPa, particularly 0.06 MPa; a feeding speed of 10-60 mL/h, particularly 30 mL/h; a receiving distance of 10-50 cm, particularly 40 cm. the feeding speed preferably being 30-60 mL/h; and a gas source of the blowing spinning being one or more selected from the group consisting of compressed air, compressed nitrogen, and compressed argon.

In the above embodiment, the electrospinning technology is preferred for the spinning. Electrospinning is performed at the conditions of: a spinning voltage of 5-15 kV, particularly 10 kV; a feeding speed of 10-60 mL/h, particularly 30-40 mL/h; a receiving distance of 10-50 cm, particularly 40-45 cm; and the feeding speed preferably being 30-60 mL/h.

In the above embodiment, the spinning preferably adopts the thread throwing technology. The thread throwing is performed at the conditions of: a spinneret rotation speed of 200-5000 r/min, particularly 500-1000 r/min; and a receiving distance of 20-100 cm, particularly 30 cm.

The present invention further seeks to protect use of the above high-entropy nitride ceramic fiber provided in the present invention in photocatalytic preparation of methane from carbon dioxide and use of the high-entropy nitride ceramic fiber in preparing methane.

Particularly, in the preparation steps of methane, the above high-entropy nitride ceramic fiber provided by the present invention is used as a catalyst.

A catalytic reaction in which the catalyst participates is photocatalysis.

In the photocatalysis, visible light is used as light source.

In the photocatalysis, raw materials comprise carbon dioxide.

The mass ratio of the catalyst to the carbon dioxide is 1:80-90, particularly 1:83. Particularly, the raw materials comprise water and carbon dioxide.

The present invention further provides use of the above high-entropy nitride ceramic fiber in photocatalytic preparation of methane from carbon dioxide. Compared with use of high-entropy nitride ceramics in the prior art, the present invention prepared the high-entropy nitride ceramic in fiber form and explored its application in photocatalysis. This is the first time that high-entropy nitride ceramic was used in preparation of CH₄ from CO₂ by photocatalytic process. The high-entropy nitride ceramic fiber has a high catalytic activity, and the catalyst and reactant products can be readily separated.

### Brief Description of Drawings

Fig. 1 is a fiber XRD pattern obtained in Example 2 of the present invention;
Fig. 2 is a fiber XRD pattern obtained in Example 3 of the present invention;
Fig. 3 is a fiber XRD pattern obtained in Example 4 of the present invention;
Fig. 4 is a fiber SEM image obtained in Example 2 of the present invention;
Fig. 5 is a fiber EDS mapping obtained in Example 3 of the present invention;
Fig. 6 is a photograph image of the fiber prepared in Example 4 of the present invention; and
Fig. 7 is a gas chromatogram after photocatalytic reaction in Example 13 of the present invention.

### Detailed Description of Embodiments

The present invention will be further described below in conjunction with particular examples. However, the present invention is not limited to the following examples. Unless otherwise specified, the methods are all conventional ones. The raw materials can all be obtained from open commercial channels unless otherwise specified.

### Example 1

This example provides a general preparation method of high-entropy ceramic precursor, in particular as follows.
(1) Obtaining metal alkoxides:
   Transition metal alkoxides comprising different types of elements are selected. When M in the metal alkoxide is Hf, Nb, Ta, or Mo, the alkoxide is prepared as follows. Metal salt MClₙ or M(NO₃)ₙ is dispersed in a solvent, into which a monohydric alcohol is added dropwise at a temperature in the range from -10 °C to 5 °C, followed by adding dropwise of triethylamine, then refluxing for 1-5 hours, and then filtration to obtain a metal alkoxide solution.
   When M in the metal alkoxide is Hf, n is 4.
   When M in the metal alkoxide is Nb, Ta, or Mo, n is 5.
   The molar ratio of the metal salt to monohydric alcohol to triethylamine is 1: (1-2)n: (1-1.5)n.
   The solvent is one or more selected from the group consisting of n-hexane, n-heptane, toluene, xylene, ethylene glycol dimethyl ether, ethylene glycol diethyl ether, and tert-butyl methyl ether.
   The monohydric alcohol is one or more selected from the group consisting of methanol, ethanol, isopropanol, n-propanol, n-butanol, isobutanol, ethylene glycol monomethylether, and ethylene glycol ethyl ether.
(2) Preparation of metal alkoxide complexes:
   Under the condition from room temperature to 80 °C, a complexing agent is added dropwise into the metal alkoxide M(OR)n selected in step (1), followed by stirring for 0.1-5 hours to prepare the metal alkoxide complex.
   The molar ratio of the metal alkoxide to the complexing agent is 1: (0.15-0.5)n.
   When M in the metal alkoxide is Ti or Hf, n is 4.
   When M in metal alkoxide is Nb, Ta, or Mo, n is 5.
   The complexing agent is one or two selected from the group consisting of acetylacetone and ethyl acetoacetate.
(3) Cohydrolysis:
   The metal alkoxide complexes comprising different metal elements prepared according to step (2) are selected and uniformly mixed, into which a mixture of water and monohydric alcohol is added dropwise at room temperature to 90 °C, wherein the molar ratio of water to total metals is 0.8-1.3:1 and the mass ratio of monohydric alcohol to water is 3-8:1, followed by refluxing for 1-5 hours, and atmospheric distillation to obtain a metal alkoxide copolymer.
   The monohydric alcohol is one or more selected from the group consisting of methanol, ethanol, isopropanol, n-propanol, n-butanol, isobutanol, ethylene glycol monomethylether, and ethylene glycol ethyl ether.
(4) Preparation of a precursor:
   The metal alkoxide copolymer prepared in step (3) is uniformly mixed with allyl phenolic, wherein the ratio of the total molar quantity of the metal elements to the mass of allyl-functional novolac resin is 1 mol: 18-20 g. The temperature is raised to 50-90 °C, and reaction is performed for 0.5-4 hours. After that, the temperature is lowered to obtain the high-entropy ceramics precursor.

### Example 2

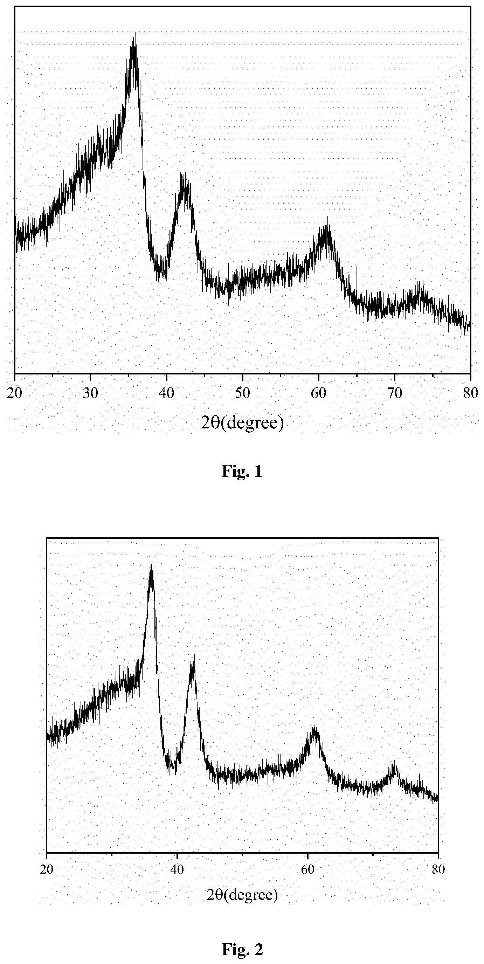

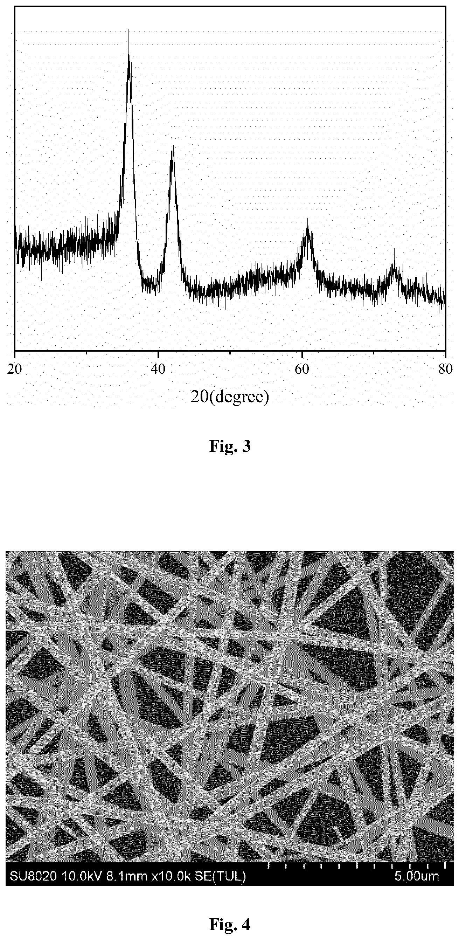

In this example, a high-entropy nitride ceramic fiber was prepared by the following method.
1. Preparation of a high-entropy precursor: the precursor was prepared according to the method of Example 1, with specific steps as follows.
   (1) Obtaining Metal alkoxides: The metal alkoxides were selected from the group consisting of Hf(Oi-Pr)₄, Ti(OPr)₄, Ta(OCH₂CH₂OCH₃)₅, Mo(OCH₂CH₂OCH₂CH₃)₅, and Nb(OPr)₅, wherein Mo(OCH₂CH₂OCH₂CH₃)₅, Hf(Oi-Pr)₄, Ta(OCH₂CH₂OCH₃)₅, and Nb(OPr)₅ were obtained as follows. Metal salts MoCl₅, HfCl₄, TaCls, and NbCl₅ were dispersed in n-heptane, respectively, into which ethylene glycol ethyl ether, isopropanol, ethylene glycol monomethylether, and n-propanol were respectively added dropwise at 0 °C, followed by respective adding dropwise of triethylamine. After that, the system was refluxed for 2 h, followed by respective filtration to obtain metal alkoxide solutions. The ratios of metal salts MoCl₅, HfCl₄, TaCls, and NbCl₅ to the monohydric alcohol to triethylamine were 1:6:5, 1:4:4, 1:10:6, and 1:6:6, respectively.
   (2) Preparation of metal alkoxide complexes:
      At 50 °C, acetylacetone was added dropwise into metal alkoxides Hf(Oi-Pr)₄, Ti(OPr)₄, Ta(OCH₂CH₂OCH₃)₅, Mo(OCH₂CH₂OCH₂CH₃)₅, and Nb(OPr)₅, respectively, followed by stirring for 1 hour.
      The molar ratios of the metal alkoxides Hf(Oi-Pr)₄, Ti(OPr)₄, Ta(OCH₂CH₂OCH₃)₅, Mo(OCH₂CH₂OCH₂CH₃)₅, and Nb(OPr)₅ to acetylacetone were 1:1.1, 1:0.8, 1:1, 1:2, and 1:1.5, respectively;
   (3) Cohydrolysis:
      The metal alkoxide complexes obtained in step (2) were uniformly mixed in an equal metal molar ratio. A mixed solution of water and n-propanol was added dropwise into the system at 70 °C, wherein the molar ratio of water to total metals was 1.2:1, and the mass ratio of n-propanol to water was 8:1. After that, refluxing was performed for 2 hours.
      A metal alkoxide copolymer was obtained by atmospheric distillation.
   (4) Preparation of a precursor:
      The metal alkoxide copolymer obtained in step (3) was uniformly mixed with allyl-functional novolac resin. The ratio of the total molar quantity of the metal elements in the alkoxide copolymer to the mass of allyl phenolic was 1 mol: 19.5 g. The temperature was raised to 80 °C and reaction was performed for 1 hour. The temperature was then lowered to obtain the high-entropy ceramics precursor.
2. Preparation of high-entropy nitride ceramic fiber
   (1) Preparation of a spinnable precursor solution:
      30 g of the high-entropy ceramic precursor, 10 g of polyvinylpyrrolidone, and 150 g of ethanol were mixed and stirred to obtain a brown homogenous solution.
   (2) Spinning and collection:
      Compressed air was used as a gas source, and the precursor solution obtained in step (1) was stretched into nanofiber by means of a blow spinning device. The spinning was performed at the pressure of 0.09 MPa, the feeding speed of 30 mL/h, and the receiving distance of 40 cm.
   (3) Pyrolyzation:
      Nanofiber collected in step (2) was placed in a heat treatment device. The temperature was raised to 600 °C at the heating rate of 1 °C /min in nitrogen atmosphere, and was kept for 2 hours to obtain a pyrolyzed fiber.
   (4) Nitriding:
      The pyrolyzed fiber prepared in step (3) was placed in a heat treatment device and nitrided in ammonia atmosphere at the temperature of 800 °C for 2 hours to obtain a high-entropy nitride fiber cotton.
      The XRD pattern of the high-entropy nitride ceramic fiber is shown in Fig. 1. As shown in the figure, the fiber forms a single crystal phase structure, indicating that the high-entropy nitride ceramic was successfully prepared. Its SEM is shown in Fig. 4. As shown in the figure, the diameter of the fiber is rather homogenous. The specific surface area of the fiber was measured to be 6 m²/g by a specific surface area analyzer.
      The ultra-high temperature performance of the high-entropy nitride ceramic fiber could not be tested in a general laboratory, because it could not melt at a temperature below 3000 °C.

### Example 3

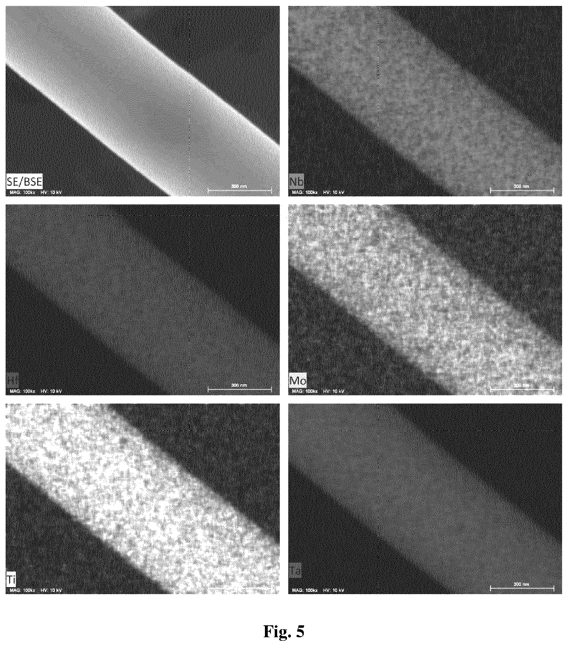

In this Example, a high-entropy nitride ceramic fiber was prepared by the following method.
(1) Preparation of a spinnable precursor solution:
   A high-entropy ceramics precursor comprising Ti, Hf, Ta, Nb, and Mo was prepared by the method recited in Example 1, and the specific preparation method was the same as that in Example 2.
   30 g of the high-entropy ceramic precursor, 10 g of polyvinyl butyral, and 285 g of n-propanol were mixed and stirred to obtain a brown homogenous solution.
(2) Spinning and collection:
   Compressed nitrogen was used as a gas source, the precursor solution obtained in step (1) was air spun by means of a blow spinning device and stretched into nanofiber at the spinning pressure of 0.06 MPa, the feeding speed of 30 mL/h, and the receiving distance of 40 cm.
(3) Pyrolyzation:
   The nanofiber collected in step (2) was placed in a heat treatment device, and the temperature was raised to 600 °C at the heating rate of 1.5 °C /min in argon atmosphere, and kept for 2 hours to obtain a pyrolyzed fiber.
(4) Nitriding:
   The pyrolyzed fiber prepared in step (3) was placed in a heat treatment device and nitrided in ammonia atmosphere at the nitriding temperature of 900 °C for 2 hours to obtain a high-entropy nitride fiber.
   The XRD pattern of the high-entropy nitride ceramic fiber is shown in Fig. 2. As shown in the figure, the fiber forms a single crystal phase structure, indicating that the high-entropy nitride ceramic has been successfully prepared. The EDS mapping of the fiber shows that all metal elements in the fiber are rather homogenous distributed (Fig. 5).
   The ultra-high temperature performance of the high-entropy nitride ceramic fiber could not be tested in a general laboratory, because it could not melt at a temperature below 3000 °C.

### Example 4

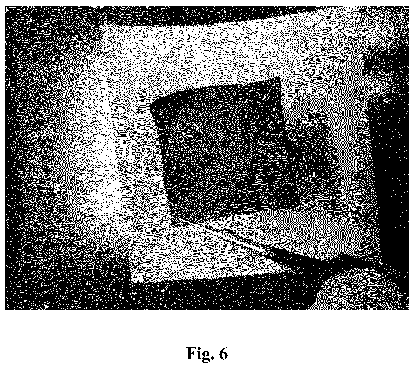

In this Example, a high-entropy nitride ceramic fiber was prepared by the following method.
1. Preparation of a high-entropy precursor: the precursor was prepared according to the method of Example 1, with specific steps as follows.
   (1) Obtaining metal alkoxides: The metal alkoxides were selected from the group consisting of Hf(OPr)₄, Ti(OPr)₄, Ta(OPr)₅, Mo(OPr)₅, and Nb(OCH₂CH₂OCH₃)₅, wherein Hf(OPr)₄, Ta(OPr)₅, Mo(OPr)₅, and Nb(OCH₂CH₂OCH₃)₅ were obtained as follows. Metal salts HfCl₄, TaCls, MoCl₅, and NbCl₅ were dispersed in n-hexane respectively, and monohydric alcohols n-propanol, n-propanol, n-propanol, and ethylene glycol monomethylether were added dropwise at -5 °C respectively, followed by respective adding dropwise of triethylamine. After that, refluxing was performed for 2 hours under heating, and the metal alkoxide solutions were obtained by respective filtration. Therein, the ratios of metal salts HfCl₄, TaCls, MoCl₅, and NbCl₅ to the monohydric alcohol to triethylamine were 1:6:5, 1:5:5, 1:10:6, and 1:8:7, respectively.
   (2) Preparation of metal alkoxide complexes:
      At room temperature, acetylacetone was added dropwise into metal alkoxides Hf(OPr)₄, Ti(OPr)₄, Ta(OPr)₅, Mo(OPr)s, and Nb(OCH₂CH₂OCH₃)₅, respectively, followed by stirring for 1 h.
      The molar ratios of metal alkoxides Hf(OPr)₄, Ti(OPr)₄, Ta(OPr)₅, Mo(OPr)s, and Nb(OCH₂CH₂OCH₃)₅ to acetylacetone were 1:0.5, 1:0.8, 1:1, 1:2, and 1:0.9, respectively.
   (3) Cohydrolysis:
      The metal alkoxide complexes obtained in step (2) were uniformly mixed in equal metal molar ratio. A mixed solution of water and n-propanol was added dropwise into the system at room temperature, wherein the molar ratio of water to total metals was 1:1, and the mass ratio of n-propanol to water was 5:1. After that, refluxing was performed for 2 hours.
      A metal alkoxide copolymer was obtained by atmospheric distillation.
   (4) Preparation of a precursor:
      The metal alkoxide copolymer obtained in step (3) was uniformly mixed with allyl-functional novolac resin. The ratio of the total molar quantity of the metal elements in the alkoxide copolymer to the mass of allyl-functional novolac resin was 1 mol: 18 g. The temperature was raised to 90 °C and reaction was performed for 3 h. The temperature was then lowered to obtain the high-entropy ceramics precursor.
2. Preparation of high-entropy nitride ceramic fiber
   (1) Preparation of a spinnable precursor solution:
      30 g of the high-entropy ceramic precursor, 10 g of polyvinyl acetate, and 290 g of ethanol were taken to be mixed and stirred to obtain a brown homogenous solution.
   (2) Spinning and collection:
      The precursor solution obtained in step (1) was stretched into nanofiber by means of an electrospinning device at the spinning voltage of 10 kV, the feeding speed of 40 mL/h, and the receiving distance of 40 cm.
   (3) Pyrolyzation:
      The nanofiber collected in step (2) was placed in a heat treatment device. The temperature was raised to 600 °C at the heating rate of 1 °C /min in argon atmosphere, and was kept for 2 hours to obtain a pyrolyzed fiber.
   (4) Nitriding:
      The pyrolyzed fiber prepared in step (3) was placed in a heat treatment device and nitrided in ammonia atmosphere at the nitriding temperature of 1000 °C for 2 hours to obtain a high-entropy nitride fiber.
      The XRD pattern of the high-entropy nitride ceramic fiber is shown in Fig. 3, and the photograph image of the fiber is shown in Fig. 6.
      The ultra-high temperature performance of the high-entropy nitride ceramic fiber could not be tested in a general laboratory, because it could not melt at a temperature below 3000 °C.

### Example 5

In this Example, a high-entropy nitride ceramic fiber was prepared by the following method.
(1) Preparation of a spinnable precursor solution:
   A high-entropy ceramics precursor comprising Ti, Hf, Ta, Nb, and Mo was prepared by the method recited in Example 1. The specific preparation method was the same as that in Example 2, but with the molar percentage ratio between metals during cohydrolysis of the metal alkoxide complexes being selected as Ti: Hf: Ta: Nb: Mo = 10:35:35:10:10.
   30 g of the high-entropy ceramic precursor, 10 g of polymethylmethacrylate, and 300 g of ethylene glycol monomethylether were mixed and stirred to obtain a brown homogenous solution.
(2) Spinning and collection:
   The precursor solution obtained in step (1) was stretched into nanofiber by means of an electrospinning device at the spinning voltage of 15 kV, the feeding speed of 30 mL/h, and the receiving distance of 45 cm.
(3) Pyrolyzation:
   The nanofiber collected in step (2) was placed in a heat treatment device. The temperature was raised to 600 °C at the heating rate of 1.5 °C /min in argon atmosphere, and kept for 2 hours to obtain a pyrolyzed fiber.
(4) Nitriding:
   The pyrolyzed fiber prepared in step (3) was placed in a heat treatment device and nitrided in ammonia atmosphere at the nitriding temperature of 800 °C for 2 hours to obtain a high-entropy nitride fiber.
   The ultra-high temperature performance of the high-entropy nitride ceramic fiber could not be tested in a general laboratory, because it could not melt at a temperature below 3000 °C.

### Example 6

In this Example, a high-entropy nitride ceramic fiber was prepared by the following method.
(1) Preparation of a spinnable precursor solution:
   A high-entropy ceramics precursor comprising Ti, Hf, Ta, Nb, and Mo was prepared by the method recited in Example 1. The specific preparation method was the same as that in Example 2, but with the molar percentage ratio between metals during cohydrolysis of the metal alkoxide complexes being selected as Ti: Hf: Ta: Nb: Mo = 15:30:35:15:5.
   30 g of the high-entropy ceramic precursor, 10 g of polyvinyl acetate, and 290 g of ethanol were mixed and stirred to obtain a brown homogenous solution.
(2) Spinning and collection:
   The precursor solution obtained in step (1) was stretched into fiber by centrifugal spinning at a rotation speed of 1000 r/min, and the receiving distance of 30 cm.
(3) Pyrolyzation:
   The nanofiber cotton collected in step (2) was placed in a heat treatment device. The temperature was raised to 600 °C at the heating rate of 1 °C /min in argon atmosphere, and kept for 2 hours to obtain a pyrolyzed fiber.
(4) Nitriding:
   The pyrolyzed fiber prepared in step (3) was placed in a heat treatment device and nitrided in ammonia atmosphere at the nitriding temperature of 1000 °C for 2 hours to obtain a high-entropy nitride fiber.
   The ultra-high temperature performance of the high-entropy nitride ceramic fiber could not be tested in a general laboratory, because it could not melt at a temperature below 3000 °C.

### Example 7

In this Example, a high-entropy nitride ceramic fiber was prepared by the following method.
(1) Preparation of a spinnable precursor solution:
   A high-entropy ceramics precursor comprising Ti, Hf, Ta, Nb, and Mo was prepared by the method recited in Example 1. The specific preparation method was the same as that in Example 4, but with the molar percentage ratio between metals during cohydrolysis of the metal alkoxide complexes being selected as Ti: Hf: Ta: Nb: Mo = 20:30:30:15:5.
   30 g of the high-entropy ceramic precursor, 2 g of polyvinyl butyral, 10 g of polyvinylpyrrolidone, and 600 g of N, N-dimethylformamide were mixed and stirred to obtain a brown homogenous solution.
(2) Spinning and collection:
   Compressed argon was used as a gas source, and the precursor solution obtained in step (1) was gas spun by means of a blow spinning device, stretched into nanofiber at the spinning pressure of 0.02 MPa, the feeding speed of 10 mL/h, and the receiving distance of 10 cm.
(3) Pyrolyzation:
   The nanofiber collected in step (2) was placed in a heat treatment device. The temperature was raised to 550 °C at the heating rate of 0.5 °C /min in nitrogen atmosphere, and kept for 4 hours to obtain a pyrolyzed fiber.
(4) Nitriding:
   The pyrolyzed fiber prepared in step (3) was placed in a heat treatment device and nitrided in ammonia atmosphere at the nitriding temperature of 600 °C for 0.5 hour to obtain a high-entropy nitride fiber.
   The ultra-high temperature performance of the high-entropy nitride ceramic fiber could not be tested in a general laboratory, because it could not melt at a temperature below 3000 °C.

### Example 8

In this Example, a high-entropy nitride ceramic fiber was prepared by the following method.
(1) Preparation of a spinnable precursor solution:
   A high-entropy ceramics precursor comprising Ti, Hf, Ta, Nb, and Mo was prepared by the method recited in Example 1. The specific preparation method was the same as that in Example 4, but with the molar percentage ratio between metals during cohydrolysis of the metal alkoxide complexes being selected as Ti: Hf: Ta: Nb: Mo = 30:5:35:15:15.
   30 g of the high-entropy ceramic precursor, 8 g of polyvinyl acetate, 7 g of polyvinyl butyral, and 185 g of n-propanol were mixed and stirred to obtain a brown homogenous solution.
(2) Spinning and collection:
   Compressed nitrogen was used as a gas source, and the precursor solution obtained in step (1) was stretched into nanofiber by means of a blow spinning device at the spinning pressure of 0.2 MPa, the feeding speed of 60 mL/h, and the receiving distance of 50 cm.
(3) Pyrolyzation:
   The nanofiber collected in step (2) was placed in a heat treatment device. The temperature was raised to 600 °C at the heating rate of 3.5 °C /min in argon atmosphere, and kept for 3 hours to obtain a pyrolyzed fiber.
(4) Nitriding:
   The pyrolyzed fiber prepared in step (3) was placed in a heat treatment device and nitrided in ammonia atmosphere at the nitriding temperature of 700 °C for 5 hours to obtain a high-entropy nitride fiber.
   The ultra-high temperature performance of the high-entropy nitride ceramic fiber could not be tested in a general laboratory, because it could not melt at a temperature below 3000 °C.

### Example 9

In this Example, a high-entropy nitride ceramic fiber was prepared by the following method.
(1) Preparation of a spinnable precursor solution:
   A high-entropy ceramics precursor comprising Ti, Hf, Ta, Nb, and Mo was prepared by the method recited in Example 1. The specific preparation method was the same as that in Example 4, but with the molar percentage ratio between metals during cohydrolysis of the metal alkoxide complexes being selected as Ti: Hf: Ta: Nb: Mo = 20:15:5:30:30.
   30 g of the high-entropy ceramic precursor, 2 g of polymethylmethacrylate, 1 g of polyvinyl acetate, and 160 g of ethanol were mixed and stirred to obtain a brown homogenous solution.
(2) Spinning and collection:
   The precursor solution obtained in step (1) was stretched into nanofiber by means of an electrospinning device at the spinning voltage of 5 kV, the feeding speed of 10 mL/h, and the receiving distance of 10 cm.
(3) Pyrolyzation:
   The nanofiber collected in step (2) was placed in a heat treatment device. The temperature was raised to 500 °C at the heating rate of 1.5 °C /min in nitrogen atmosphere, and kept for 2 hours to obtain a pyrolyzed fiber.
(4) Nitriding:
   The pyrolyzed fiber prepared in step (3) was placed in a heat treatment device and nitrided in ammonia atmosphere at the nitriding temperature of 1000 °C for 2.5 hours to obtain a high-entropy nitride fiber.
   The ultra-high temperature performance of the high-entropy nitride ceramic fiber could not be tested in a general laboratory, because it could not melt at a temperature below 3000 °C.

### Example 10

In this Example, a high-entropy nitride ceramic fiber was prepared by the following method.
(1) Preparation of a spinnable precursor solution:
   A high-entropy ceramics precursor comprising Ti, Hf, Ta, Nb, and Mo was prepared by the method recited in Example 1. The specific preparation method was the same as that in Example 4, but with the molar percentage ratio between metals during cohydrolysis of the metal alkoxide complexes being selected as Ti: Hf: Ta: Nb: Mo = 8:12:30:15:35.
   30 g of the high-entropy ceramic precursor, 15 g of polymethylmethacrylate, 10 g of ethanol, and 270 g of ethylene glycol monomethylether were mixed and stirred to obtain a brown homogenous solution.
(2) Spinning and collection:
   The precursor solution obtained in step (1) was stretched into nanofiber by means of an electrospinning device at the spinning voltage of 10kV, the feeding speed of 60 mL/h, and the receiving distance of 50 cm.
(3) Pyrolyzation:
   The nanofiber collected in step (2) was placed in a heat treatment device. The temperature was raised to 600 °C at the heating rate of 2 °C /min in helium atmosphere, and kept for 3 hours to obtain a pyrolyzed fiber.
(4) Nitriding:
   The pyrolyzed fiber prepared in step (3) was placed in a heat treatment device and nitrided in ammonia atmosphere at the nitriding temperature of 800 °C for 2 hours to obtain a high-entropy nitride fiber.
   The ultra-high temperature performance of the high-entropy nitride ceramic fiber could not be tested in a general laboratory, because it could not melt at a temperature below 3000 °C.

### Example 11

In this Example, a high-entropy nitride ceramic fiber was prepared by the following method.
(1) Preparation of a spinnable precursor solution:
   A high-entropy ceramic precursor comprising Ti, Hf, Ta, Nb, and Mo was prepared by the method recited in Example 1. The specific preparation method was the same as that in Example 4, but with the molar percentage ratio between metals during cohydrolysis of the metal alkoxide complexes being selected as Ti: Hf: Ta: Nb: Mo = 10:10:30:30:20.
   30 g of the high-entropy ceramic precursor, 10 g of polyvinyl butyral, 130 g of n-propanol, and 60 g of acetone were mixed and stirred to obtain a brown homogenous solution.
(2) Spinning and collection:
   The precursor solution obtained in step (1) was stretched into fiber by centrifugal spinning at a rotation speed of 500 r/min and the receiving distance of 20 cm.
(3) Pyrolyzation:
   The nanofiber collected in step (2) was placed in a heat treatment device. The temperatures was raised to 600 °C at the heating rate of 1.5 °C /min in nitrogen atmosphere, and kept for 2 hours to obtain a pyrolyzed fiber.
(4) Nitriding:
   The pyrolyzed fiber prepared in step (3) was placed in a heat treatment device and nitrided in ammonia atmosphere at the nitriding temperature of 900 °C for 2 hours to obtain a high-entropy nitride fiber.
   The ultra-high temperature performance of the high-entropy nitride ceramic fiber could not be tested in a general laboratory, because it could not melt at a temperature below 3000 °C.

### Example 12

In this Example, a high-entropy nitride ceramic fiber was prepared by the following method.
(1) Preparation of a spinnable precursor solution:
   A high-entropy ceramic precursor comprising Ti, Hf, Ta, Nb, and Mo was prepared by the method recited in Example 1. The specific preparation method was the same as that in Example 2, but with the molar percentage ratio between metals during cohydrolysis of the metal alkoxide complexes being selected as Ti: Hf: Ta: Nb: Mo = 30:5:35:15:15.
   30 g of the high-entropy ceramic precursor, 10 g of polyvinyl butyral, and 190 g of ethylene glycol monomethylether were mixed and stirred to obtain a brown homogenous solution.
(2) Spinning and collection:
   The precursor solution obtained in step (1) was stretched into nanofiber by a centrifugal spinning at a rotation speed of 5000 r/min and the receiving distance of 100 cm.
(3) Pyrolyzation:
   The nanofiber collected in step (2) was placed in a heat treatment device. The temperature was raised to 600 °C at the heating rate of 2 °C /min in N2 atmosphere, and kept for 2 hours to obtain a pyrolyzed fiber.
(4) Nitriding:
   The pyrolyzed fiber prepared in step (3) was placed in a heat treatment device and nitrided in ammonia atmosphere at the nitriding temperature of 1000 °C for 2 hours to obtain a high-entropy nitride fiber.
   The ultra-high temperature performance of the high-entropy nitride ceramic fiber could not be tested in a general laboratory, because it could not melt at a temperature below 3000 °C.

### Example 13

This example was mainly to illustrate the catalytic effect of the high-entropy nitride ceramic fiber prepared by the present invention. In the process of preparing CH₄ from CO₂, pure water and pure carbon dioxide gas were used as raw materials and the high-entropy nitride ceramic nanofiber prepared in Example 4 were used as catalyst. A photocatalytic reaction was carried out under the irradiation of a 300W Xe lamp. The mass ratio of the catalyst to carbon dioxide was 1:83. After reacting for 12 h, the gas in the reaction vessel was detected by gas chromatography. It was found that conversion products of carbon dioxide were generated, with the main product being methane, indicating that the catalyst had high catalytic selectivity.

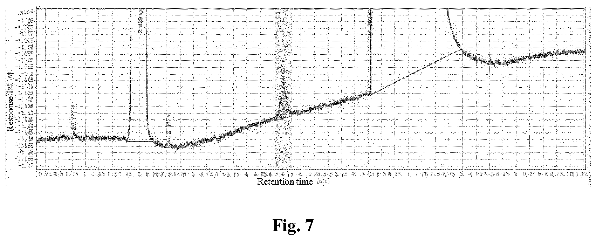

Fig. 7 shows the gas chromatography, with peak positions at 0.777 being generated H₂, 2.543 being generated CO, and 4.685 being generated CH₄. As can be seen, the photocatalysis product was mainly CH₄, and the catalytic selectivity was higher than 90%.

### Industrial Application

After adopting the above technical solution, the present invention has the following beneficial effects compared with the prior art.
1. The present invention takes a high-entropy ceramics polymer precursor comprising Ti, Hf, Ta, Nb, and Mo with the molar quantity of each of the metal elements accounting for 5-35% of the total molar quantity of the metal elements as a metal source, and employs blowing spinning, electro spinning, or centrifugal spinning as a forming means to prepare high-entropy nitride ceramic fiber. The fiber has the characteristics of homogenous diameter, high specific surface area, etc. The existing forms of high-entropy nitride ceramics are expanded.
2. The high-entropy ceramic precursor spinning solution provided by the present invention has the characteristic of adjustable rheology, and while improving the spinning performance, also enables the spinning solution to be hermetically stored at room temperature for more than 3 weeks with a viscosity change rate not exceeding 5%. This reduces restrictions on subsequent procedures (spinning, Pyrolyzation, and nitriding) and further improves spinning efficiency.
3. The present invention prepare the high-entropy ceramic fiber by blowing spinning, electro spinning, or centrifugal spinning, which requires simple equipment, convenient operations, and low costs. Continuous fiber cotton or fiber non-woven fabric with controllable average diameters can be obtained, and rapid scale-up production can be achieved.
4. In preparation of CH₄ from CO₂ by photocatalytic conversion, the high-entropy nitride fiber prepared by the present invention is characterized by high conversion efficiency, requiring no cocatalyst, easy separation of the catalyst from raw material and products, etc. This is the first time that the high-entropy nitride ceramics are used in the field and explores a new development direction for use of the high-entropy nitride ceramics.

## Claims

1. A high-entropy nitride ceramic fiber, wherein the high-entropy ceramic fiber comprises Ti, Hf, Ta, Nb, and Mo, wherein the high-entropy nitride ceramic fiber is in single crystal phase, and wherein each of the elements are uniformly distributed at molecular level.

2. The high-entropy nitride ceramic fiber according to claim 1, wherein a molar quantity of each of the metal elements in the high-entropy ceramic fiber occupies 5-35% of the total molar quantity of the metal elements; and preferably, the respective metal elements are equimolar.

3. The high-entropy nitride ceramic fiber according to claim 1, wherein the high-entropy ceramic fiber further comprises nitrogen; and wherein the molar quantity of nitrogen is the same as the total molar quantity of Ti, Hf, Ta, Nb, and Mo.

4. The high-entropy nitride ceramic fiber according to any one of claims 1-3, wherein the high-entropy ceramic fiber further comprises nitrogen and a very small amount of oxygen; and wherein the molar quantity of nitrogen is the same as the total molar quantity of Ti, Hf, Ta, Nb, and Mo.

5. A preparation method of the high-entropy nitride ceramic fiber according to any one of claims 1-4, wherein the preparation method comprises: mixing a high-entropy ceramic precursor of Ti, Hf, Ta, Nb, and Mo, a spinning aid, and a solvent uniformly to prepare a precursor spinning solution, followed by spinning, pyrolyzation, and nitriding procedures to prepare the high-entropy nitride ceramic fiber.

6. The preparation method of the high-entropy nitride ceramic fiber according to claim 5, wherein the high-entropy ceramic precursor is prepared by:
step (1) obtaining metal alkoxide complexes:
adding dropwise a complexing agent into metal alkoxides M(OR)ₙ which comprise target metal elements, followed by stirring for 0.1-5 hours to obtain the metal alkoxide complexes;
step (2) cohydrolysis:
selecting and uniformly mixing the metal alkoxide complexes which comprise different metal elements prepared according to step (1), into which a mixture of water and a monohydric alcohol is added dropwise, followed by refluxing for 1-5 hours, and atmospheric distillation to obtain a metal alkoxide copolymer;
step (3) preparing the precursor:
mixing the metal alkoxide copolymer prepared in step (2) with allyl-functional novolac resin uniformly, raising the temperature to 50-90 °C, and lowering the temperature after 0.5-4 hours of reaction to obtain the high-entropy ceramic precursor.

7. The preparation method of the high-entropy nitride ceramic fiber according to claim 6, wherein in step (1), the molar ratio of the metal alkoxide to the complexing agent is 1: (0.15-0.5) n; wherein the complexing agent is acetylacetone and/or ethyl acetoacetate; wherein in M(OR)ₙ of step (1): when M is Ti or Hf, n is 4; when M is Nb, Ta, or Mo, n is 5; and R is at least one selected from the group consisting of a C1-C6 alkyl and a C1-C6 alkoxy, particularly at least one selected from the group consisting of C1-C4 alkyl and C1-C4 alkoxy, and more particularly at least one selected from the group consisting of ethyl, ethylene glycol diethyl ether, i-Pr, -Pr, and -CH₂CH₂OCH₃.

8. The preparation method of the high-entropy nitride ceramic fiber according to claim 6 or 7, wherein in the precursor spinning solution, the mass ratio of the high-entropy ceramic precursor to the spinning aid to the solvent is 1:0.1-1:5-20, preferably 1:0.2-0.5:5-10.

9. The preparation method of the high-entropy nitride ceramic fiber according to any one of claims 5-8, wherein in step (2), the molar ratio of water to the total metal is 0.8-1.3:1, and the mass ratio of the monohydric alcohol to water is 3-8:1; and wherein the monohydric alcohol is at least one selected from the group consisting of methanol, ethanol, isopropanol, n-propanol, n-butanol, isobutanol, ethylene glycol monomethylether, and ethylene glycol ethyl ether.

10. The preparation method of the high-entropy nitride ceramic fiber according to any one of claims 5-9, wherein in step (3), the ratio of a total molar quantity of the metal elements in the metal alkoxide copolymer to the mass of allyl-functional novolac resin is 1 mol: 18-20 g.

11. The preparation method of the high-entropy nitride ceramic fiber according to any one of claims 5-10, wherein the nitriding comprises: nitriding the pyrolyzed fiber in ammonia atmosphere at a temperature in the range from 600 to 1000 °C for a period in the range from 0.5 to 5 hours.

12. The preparation method of the high-entropy nitride ceramic fiber according to any one of claims 5-11, wherein the spinning aid is at least one selected from the group consisting of polymethylmethacrylate, polyvinyl acetate, polyvinyl butyral, and polyvinylpyrrolidone; and wherein the solvent is at least one selected from the group consisting of ethanol, acetone, n-propanol, ethylene glycol monomethylether, and N, N-dimethylformamide.

13. The preparation method of the high-entropy nitride ceramic fiber according to any one of claims 5-12, wherein the pyrolyzation comprises: raising the temperature to 500-600 °C at a heating rate of 0.5-5 °C /min in an inert atmosphere, and maintaining the temperature for 2-4 hours.

14. The preparation method of the high-entropy nitride ceramic fiber according to any one of claims 5-13, wherein the spinning is at least one selected from the group consisting of blowing spinning, electro spinning, and centrifugal spinning.

15. Use of the high-entropy nitride ceramic fiber according to any one of claims 1-4 in preparation of methane from carbon dioxide by photocatalysis.

16. Use of the high-entropy nitride ceramic fiber according to any one of claims 1-4 in preparation of methane.

17. The use according to claim 16, wherein a catalyst used in the preparation of methane is the high-entropy nitride ceramic fiber according to any one of claims 1-4.

18. The use according to claim 16 or 17, wherein a catalytic reaction in which the catalyst participates is photocatalysis; wherein in the photocatalysis, a light source used is visible light; wherein in the photocatalysis, a raw material comprises carbon dioxide; and particularly, the raw material comprises water and carbon dioxide.
